# FASCICULE DE BREVET EUROPEEN

(11) **EP 2 482 851 B1**
(45) Date de publication et mention de la délivrance du brevet: **11.11.2020**
(21) Numéro de dépôt: 10773649.8
(22) Date de dépôt: 27.09.2010
(51) Int. Cl.: A61K 47/12, A61K 31/167, A61K 31/192, A61K 9/08

(54) **FORMULATION LIQUIDE, STABLE, PRETE A L'EMPLOI DE KETOPROFENE**
STABILE, FLÜSSIGE, GEBRAUCHSFERTIGE KETOPROFENFORMULIERUNG
STABLE, LIQUID, READY-TO-USE KETOPROFEN FORMULATION

(30) Priorité: 28.09.2009 FR 0904622
(43) Date de publication de la demande: 08.08.2012
(73) Titulaire: Maco Pharma, 59420 Mouvaux (FR)
(72) Inventeur: DOULEAU, Didier, 33115 Pyla sur Mer (FR); RENARD, Julien, 59130 Lambersart (FR)
(74) Mandataire: Sayettat, Julien Christian
(86) Numéro de dépôt international: PCT/FR2010/000641
(87) Numéro de publication internationale: WO 2011/036357

(56) Documents cités:
- EP-A1- 2 087 909
- EP-B1- 0 814 797
- WO-A1-2004/037242
- US-A- 6 028 222

## Description

L'invention concerne une formulation liquide, stable, prête-à-l'emploi de kétoprofène, en particulier pour l'administration par voie parentérale, notamment par voie intraveineuse comme décrit dans la revendication 1.

L'invention s'applique notamment à l'administration du kétoprofène par perfusion pour traiter les douleurs post-opératoires et les crises de coliques néphrétiques.

Il est connu depuis très longtemps d'utiliser du kétoprofène sous forme racémique ou énantiomère comme médicament antalgique, anti-inflammatoire et antipyrétique bien toléré par l'homme. En effet, le kétoprofène est l'un des médicament anti-inflammatoire non stéroïdien (AINS) les plus actifs. Il fait partie des dérivés de l'acide propionique ayant une activité anti-inflammatoire et analgésique rapide.

Le kétoprofène possède une très faible solubilité dans l'eau (52 mg/L) et dans les solutions acides. Cette pauvre solubilité s'explique par la présence d'un groupement aromatique lipophile. Le kétoprofène est plus soluble dans les solutions basiques mais se dégrade ensuite rapidement, démontrant l'instabilité de telles solutions basiques.

En France, il existe une seule spécialité injectable par voie intraveineuse, commercialisée sous le nom de Profenid®. La spécialité se présente sous la forme d'un flacon de lyophilisat contenant 100 mg de kétoprofène, de l'hydroxyde de sodium, de la glycine, et de l'acide citrique. Le lyophilisat doit être dissout extemporanément dans une solution isotonique de glucose ou de chlorure de sodium avant injection au patient.

Mais, la solution de kétoprofène ainsi reconstituée présente une osmolarité supérieure à celle du plasma et provoque des douleurs lors de l'injection au patient. L'ajustement de l'osmolarité n'est plus possible après la reconstitution.

De plus, la reconstitution de la solution médicamenteuse nécessite du matériel médical supplémentaire, tel que des dispositifs de transfert, des lignes de perfusion et des poches de solvant. Elle est génératrice de temps pour le personnel médical et provoque, le temps de reconstituer la solution, un retard dans le traitement de la douleur d'un patient.

Afin de surmonter les problèmes de solubilité du kétoprofène, plusieurs méthodes ont été proposées, parmi lesquelles l'utilisation de co-solvant ou la formation de complexes ou de sels azotés de kétoprofène. Les solutions aqueuses de tels complexes ou sels azotés présentent encore un pH supérieur ou égal à 7.

Dans l'article Singhai. et al, Pharmazie 52:226-228 (1997), il est indiqué que la solubilité et la stabilité du kétoprofène dans l'eau sont augmentées à l'aide des co-solvants polyéthylène glycol et propylène glycol, ou du benzoate de sodium. Mais le polyéthylène glycol n'est pas recommandé pour une administration par voie intraveineuse du fait de sa toxicité.

Le document US-5 895 789 propose une solution aqueuse pour administration parentérale comprenant un sel alkylammonium de kétoprofène, par exemple le sel L-lysine de kétoprofène, la solution ayant un pH entre 7 et 7,5 et étant exempte de conservateur, de co-solvants et de substances de support tel que la glycine. Mais cette solution doit être fabriquée et conservée dans une atmosphère de gaz inerte qui nécessite des installations particulières.

De même, le document WO-99/52528 décrit des sels hydrosolubles de kétoprofène obtenus par réaction du kétoprofène avec la glucosamine, la proline et/ou l'hydroxyproline et utilisés dans des préparations injectables, des comprimés ou des gels.

Il a également été envisagé dans le document WO-2006/116626 de former des complexes de kétoprofène avec la pipérazine ou l'éthylènediamine pour augmenter la solubilité du kétoprofène et réduire l'irritation lors d'une administration parentérale. Le pH des solutions contenant ces complexes est compris entre 6,5 et 8,5.

Enfin WO 2004/037242 décrit des compositions liquides de Kétoprofène visant la stabilité et l'absence de douleurs à l'injection moyennant un pH entre 8 et 9.

Le but de la présente invention est de fournir des formulations stables prêtes à l'emploi de kétoprofène pour administration parentérale.

La demanderesse a constaté qu'en utilisant une solution uniquement aqueuse comprenant un agent tampon , composé d'acide acétique et d'acétate de sodium et en fixant le pH de la solution aux alentours de 6, le kétoprofène est solubilisé sans nécessité la présence d'un co-solvant non aqueux. De plus, ces solutions aqueuses sont stables dans le temps.

A cet effet et selon un premier aspect, l'invention concerne une formulation liquide stable prête-à-l'emploi de kétoprofène pour administration par voie parentérale comprenant du kétoprofène solubilisé dans un solvant uniquement aqueux additionné d'un agent tampon, composé d'acide acétique et d'acétate de sodium, ladite formulation étant ajustée à un pH supérieur à 5,5 et inférieur à 6,5.

Selon un autre aspect de l'invention, la formulation de kétoprofène est comprise dans une poche stérile.

Selon un autre aspect, l'invention se rapporte à un procédé de préparation d'une formulation selon le premier aspect.

L'invention sera comprise grâce à la description qui suit.

Selon un premier aspect, l'invention consiste en une formulation liquide stable prête-à-l'emploi de kétoprofène pour administration par voie parentérale, notamment sous forme d'une injection par voie intraveineuse pour un usage analgésique et ou anti-inflammatoire.

La formulation est prête-à-l'emploi, c'est-à-dire qu'elle est prête à être administrée directement à un patient, sans nécessiter d'étape de traitement tel qu'une reconstitution ou une dilution. Cette formulation offre donc un gain de temps appréciable pour le personnel médical, ainsi que la certitude d'administrer la bonne formulation du médicament.

Cette formulation comprend comme principe actif le kétoprofène (acide 3-benzoyl-α-methylbenzèneacétique) sous forme racémique ou l'un de ses isomère R ou S.

Le principe actif n'est pas complexé, notamment avec un composé tel que la pipérazine ou l'éthylènediamine.

La formulation comprend notamment entre 0,01 et 1 % en poids de kétoprofène, notamment 0,1% en poids.

Selon l'invention, la formulation comprend du kétoprofène solubilisé dans un solvant uniquement aqueux additionné d'un agent tampon , composé d'acide acétique et d'acétate de sodium, et la formulation est ajustée à un pH supérieur à 5,5 et inférieur à 6,5.

Le mélange de solvant uniquement aqueux avec l'agent tampon, composé d'acide acétique et d'acétate de sodium, constitue un milieu de solubilisation qui a pour fonction de mettre le kétoprofène en solution liquide. Un exemple particulier de milieu de solubilisation contient de l'eau pour préparations injectables et un agent tampon.

La formulation est exempte de co-solvant, notamment de solvant non aqueux. En particulier, elle ne comprend pas de glycol ou de polyol tel que le polyéthylène glycol ou le propylène glycol.

Elle ne comprend pas non plus des co-solvants non aqueux de type alcool tels que l'alcool benzylique, l'éthanol ou le glycérol.

De façon avantageuse, la solution est exempte de tout autre agent solubilisant tel que les agents tensioactifs (copolymère de polyoxypropylène/polyoxyéthylène), l'arginine, la N-méthylglucamine, la glycine, la glucosamine, la proline, l'hydroxyproline ou les composés alkylammonium tel que la lysine.

Par conséquent, dans la formulation selon l'invention, le kétoprofène n'est pas sous forme complexée ou sous forme de sel organique, notamment azoté.

En effet, la solubilisation du kétoprofène est assurée par l'agent tampon. L'agent tampon composé d'acide acétique et d'acétate de sodium, maintient approximativement le même pH malgré l'addition de petites quantités d'un acide, d'une base ou d'une dilution à la formulation.

La quantité d'agent tampon est comprise entre 0,01 et 2 % en poids, notamment 1,2 % en poids. Cette quantité varie selon la nature de l'agent tampon.

Le tampon est à base d'acétate tel que l'acétate d'un sel alcalin ou alcalinoterreux, l'acétate de sodium.

En général, les tampons sont composés d'un acide faible et de sa base conjuguée.

Le tampon est un couple acétate / acide acétique, par exemple acétate de sodium / acide acétique.

Selon une réalisation particulière, le rapport entre l'acide acétique et l'acétate de sodium est compris entre 1:20 et 1:500, notamment entre 1:60 et 1:100.

La formulation de kétoprofène selon l'invention comme revendiqué, présente un pH compris entre 5,5 et 6,5, notamment entre 5,8 et 6,3, par exemple 6. Ce pH est adapté pour l'injection de la formulation par voie intraveineuse.

La formulation tamponnée à ce pH est stable, c'est-à-dire que le kétoprofène ne se dégrade pas et sa concentration est maintenue substantiellement constante au cours du temps. Notamment, la solution est stable pendant une période supérieure à 3 mois, avantageusement supérieure à un an.

Le tampon acétate et en ajustant la solution à un pH autour de 6, la solution de kétoprofène est stable. Elle ne nécessite pas l'ajout d'un conservateur ou d'un agent antioxydant tel que le gallate de propyle ou le métabisulfite de sodium.

Selon une réalisation particulière, la formulation comprend au moins un ajusteur de pH tel que l'hydroxyde de sodium ou l'acide chlorhydrique pour ajuster le pH aux alentours de 6.

L'agent tampon est constitué de l'acide acétique / acétate de sodium, il est avantageux d'utiliser de l'acide acétique supplémentaire comme ajusteur de pH.

L'administration parentérale des solutions exige que celles-ci soient ajustées à l'isotonie. L'isotonie de la composition peut être obtenue par ajout d'une quantité judicieusement calculée de chlorure de sodium, de glucose, de mannitol, de sorbitol, de chlorure de potassium ou de chlorure de calcium, l'isotonisant souvent préféré étant le chlorure de sodium.

L'isotonie de la solution est comprise entre 270 et 330 mOsm/kg, notamment entre 280 et 290 mOsm/kg.

L'agent tampon acide acétique / acétate de sodium permet non seulement d'obtenir une solution tamponnée à un pH voisin de 6, mais aussi d'obtenir une isotonie proche de celle du plasma. Dans ce cas, il n'est pas nécessaire d'ajouter d'agent isotonisant.

Selon une réalisation particulière de l'invention, la formulation ne comprend pas d'agent isotonisant tel que le glucose ou le chlorure de sodium.

On décrit maintenant un procédé de préparation d'une formulation de kétoprofène selon le premier aspect de l'invention.

La préparation de la formulation comprend :
- une étape de dissolution du kétoprofène dans de l'eau pour préparations injectables additionné de l'agent tampon, composé d'acide acétique et d'acétate de sodium, et
- une étape d'ajustement du pH à une valeur comprise entre 5,5 et 6,5.

Selon une réalisation, la formulation est préparée à partir du kétoprofène base, c'est-à-dire sous forme d'acide libre.

Le pKa du kétoprofène est de l'ordre de 4,6. La formulation prête à l'emploi possède un pH compris entre 5,5 et 6,5. Par conséquent, le kétoprofène dans la formulation se salifie en partie.

Lorsque le tampon est à base d'un sel alcalin d'acétate, le sel alcalin de kétoprofène se forme. Dans la formulation finale, le kétoprofène est présent sous forme de sel et de base.

Selon l'invention l'agent tampon est un couple à base d'acide acétique et d'acétate, le procédé de préparation comprend les étapes :
- dissoudre le kétoprofène dans l'eau contenant l'acétate,
- additionner l'acide acétique pour obtenir une solution aqueuse stable de kétoprofène et ajuster le pH aux alentours de 6.

Lors de la première étape, la solution comprenant l'eau et la base du tampon est basique, permettant la dissolution du kétoprofène. Avec l'addition du kétoprofène et de l'acide du tampon, la solution devient acide et se stabilise.

Après l'ajout des constituants de la formulation et ajustement du pH, la formulation est stérilisée par filtration stérilisante ou chauffage.

Le conditionnement de la formulation est ensuite réalisé dans des poches souples ou des flacons à l'abri de la lumière. Les poches sont par exemple réalisées en polyéthylène, polypropylène ou polychlorure de vinyle.

Selon un autre aspect, l'invention concerne une poche stérile comprenant une formulation liquide prête à l'emploi stable de kétoprofène.

Afin de protéger la formulation de la lumière, les poches contenant le kétoprofène sont suremballées dans un emballage au moins partiellement opaque à la lumière comme par exemple des poches en aluminium ou un emballage constitué à 90% d'aluminium et à 10% de plastique imperméable aux UV. Dans un autre exemple, l'emballage est sous forme d'une poche comprenant une paroi opaque en aluminium et une paroi plastique transparente. En variante, l'emballage opaque comprend une fenêtre non opaque.

Selon une réalisation particulière, l'épaisseur du film d'aluminium constituant le suremballage est compris entre 5 et 25 µm, notamment 9 et 20 µm.

Avantageusement, le kétoprofène est également protégé de la lumière pendant sa fabrication. Par exemple, pendant la fabrication, les poches sont protégées dans un suremballage imperméable aux ultraviolets.

Selon un mode de réalisation, la formulation de kétoprofène selon l'invention comprend un autre composé analgésique et/ou anti-inflammatoire, comme par exemple le paracétamol.

Dans ce cas, le paracétamol étant très sensible à l'oxygène, la formulation contient en particulier une teneur en oxygène inférieure à 0,2 ppm, notamment inférieure à 0,1 ppm, pour éviter la dégradation du paracétamol.

Pour ce faire, la formulation subit par exemple un barbotage à l'aide d'un gaz inerte tel que l'azote ou un traitement à la vapeur blanche.

En outre, pour éviter la dégradation du paracétamol, il est avantageux de disposer entre la poche contenant la formulation et le suremballage, un absorbeur d'oxygène. L'absorbeur d'oxygène comprend par exemple une poudre de fer ou un oxyde de fer

### Exemple

| | **Solution A** | **Solution B** |
|---|---|---|
| Kétoprofène | 100 mg | 100 mg |
| Paracétamol | - | 1 g |
| Acide acétique glacial | 14,2 mg | 3,15 mg |
| Acétate de sodium trihydraté | 1910 mg | 1490 mg |
| (eq. anhydre) | (1151 mg) | (900 mg) |
| Hydroxyde de sodium | qsp pH=6 | qsp pH=6 |
| Eau ppi QSP | 100 mL | 100 mL |

La solution A est préparée comme suit :
a) introduire le kétoprofène et l'acétate de sodium sous forme de poudre dans 80 à 90% du volume final d'eau pour préparations injectables (ppi);
b) ajouter l'acide acétique glacial et ajuster le pH de la solution aux alentours de 6,
c) compléter le volume final du mélange avec de l'eau ppi;
d) faire passer la solution de kétoprofène au travers d'un filtre stérilisant;
e) remplir la solution de kétoprofène dans une poche souple, et
f) enfermer de manière étanche ladite poche dans un suremballage opaque à la lumière.

Selon cette formulation, le pH obtenu est voisin de 6. Il correspond à un pH optimal pour la perfusion et la stabilité du kétoprofène en solution aqueuse.

En outre, cette formulation présente une osmolarité de 287 mOsm/kg, qui la rend adaptée à une administration par voie intraveineuse sans ajout d'agent isotonisant.

### Etude de stabilité

Trois lots de 50 L de solution A ont été conditionnés en poches souples de 100 ml constituées à partir d'un film multi-couches à base de polypropylène. Chaque poche souple est emballée en sac aluminium.

Les lots ont été placés en conditions de stabilités accélérées, à savoir soit à une température de + 40°C et une humidité relative inférieure à 20%, soit à une température de + 55°C.

Les analyses suivantes ont été réalisées après 1 mois, 2 mois, 3 mois :
- Caractères organoleptiques
- Limpidité
- Coloration
- pH
- Dosage du kétoprofène
- Dosage des Impuretés du Kétoprofène

Après trois mois de stockage à + 40°C et 20 % d'humidité relative ou à + 55°C, les échantillons des lots étudiés présentent les caractéristiques d'une bonne stabilité : l'aspect des solutions est inchangé et les essais de limpidité et coloration demeurent conformes à la Pharmacopée Européenne, le pH reste stable à 6 et les taux d'impuretés obtenus n'augmentent pas avec la température au cours de la conservation.

Par conséquent, cette étude démontre la stabilité de la formulation de kétoprofène sans ajout d'un conservateur ou d'un antioxydant.

La préparation de la solution B est identique à celle de la solution A, avec l'étape supplémentaire d'introduction du paracétamol sous forme de poudre dans l'étape a) de la préparation des solutions A.

La présence de paracétamol dans la solution modifie les quantités d'acide acétique et d'acétate de sodium pour obtenir un pH voisin de 6. Il est à noter que, sans acide acétique, le pH de la solution est de 6,11. La quantité d'acide acétique à ajouter pour obtenir un pH de 6 est donc minime.

Dans ces conditions, l'osmolarité calculée de la solution est de 290 mOsm/Kg et ne nécessite pas l'ajout d'agent isotonisant.

Pour éviter l'oxydation du paracétamol, la préparation des solutions contenant le paracétamol est réalisée de sorte à maintenir la quantité d'oxygène dissous dans la solution en dessous de 0,2 ppm, avantageusement en dessous de 0,1 ppm.

Pour cela, on élimine l'oxygène dissous de la solution par exemple par barbotage dans la solution d'un gaz inerte tel que l'azote ou par traitement à la vapeur blanche, ou par mise sous vide suivie de la mise en place d'un ciel d'azote au dessus de la solution.

## Revendications

1. Formulation liquide stable prête-à-l'emploi de kétoprofène pour administration par voie parentérale comprenant du kétoprofène solubilisé dans un solvant uniquement aqueux additionné d'un agent tampon composé d'acide acétique et d'acétate de sodium, ladite formulation étant ajustée à un pH supérieur à 5,5 et inférieur à 6,5, la quantité de kétoprofène étant comprise entre 0,01 et 1 % en poids.

2. Formulation selon la revendication 1, **caractérisée en ce que** la quantité de kétoprofène est de 0,1% en poids.

3. Formulation selon la revendication 1 ou 2, **caractérisée en ce que** la quantité d'agent tampon est comprise entre 0,01 et 2 % en poids, notamment 1,2 % en poids.

4. Formulation selon l'une quelconque des revendication 1 à 3, **caractérisée en ce que** le rapport en poids entre l'acide acétique et l'acétate de sodium est compris dans la gamme 1:20 à 1:500.

5. Formulation selon l'une quelconque des revendications 1 à 4, **caractérisée en ce que** le solvant uniquement aqueux est de l'eau pour préparations injectables.

6. Formulation selon l'une quelconque des revendications 1 à 5, **caractérisée en ce qu'**elle comprend en outre au moins un agent ajusteur de pH.

7. Formulation selon l'une quelconque des revendications 1 à 6, **caractérisée en ce qu'**elle présente un pH de 6.

8. Formulation selon l'une quelconque des revendications 1 à 7, **caractérisée en ce qu'**elle présente une osmolarité comprise entre 270 et 330 mOsm/kg.

9. Formulation selon l'une quelconque des revendications 1 à 8, **caractérisée en ce qu'**elle se présente sous la forme d'une formulation pour injection par voie intraveineuse.

10. Formulation selon l'une quelconque des revendications 1 à 9, **caractérisée en ce qu'**elle comprend un autre composé analgésique et/ou anti-inflammatoire.

11. Formulation selon la revendication 10, **caractérisée en ce que** le composé analgésique est le paracétamol.

12. Poche stérile comprenant une formulation selon l'une quelconque des revendications 1 à 11.

13. Procédé de préparation d'une formulation selon l'une quelconque des revendications 1 à 11, **caractérisé en ce qu'**il comprend :
- une étape de dissolution du kétoprofène dans de l'eau pour préparations injectables additionné d'un agent tampon, et
- une étape d'ajustement du pH à une valeur comprise entre 5,5 et 6,5.

14. Procédé de préparation selon la revendication 13, **caractérisé en ce qu'**il comprend en outre une étape de stérilisation de la formulation obtenue après l'étape d'ajustement du pH.

## Patentansprüche

1. Stabile gebrauchsfertige flüssige Ketoprofen-Formulierung zur parenteralen Verabreichung, die in einem rein wässrigen Lösungsmittel, dem ein aus Essigsäure und Natriumacetat bestehendes Puffermittel zugegeben wurde, solubilisiertes Ketoprofen umfasst, wobei die Formulierung auf einen pH-Wert von größer als 5,5 und kleiner als 6,5 eingestellt ist, wobei die Ketoprofen-Menge zwischen 0,01 und 1 Gewichts-% liegt.

2. Formulierung nach Anspruch 1, **dadurch gekennzeichnet, dass** die Ketoprofen-Menge 0,1 Gewichts-% beträgt.

3. Formulierung nach Anspruch 1 oder 2, **dadurch gekennzeichnet, dass** die Puffermittel-Menge zwischen 0,01 und 2 Gewichts-% liegt, insbesondere 1,2 Gewichts-% beträgt.

4. Formulierung nach einem der Ansprüche 1 bis 3, **dadurch gekennzeichnet, dass** das Gewichtsverhältnis zwischen der Essigsäure und dem Natriumacetat im Bereich von 1:20 bis 1:500 liegt.

5. Formulierung nach einem der Ansprüche 1 bis 4, **dadurch gekennzeichnet, dass** es sich bei dem rein wässrigen Lösungsmittel um Wasser für Injektionszwecke handelt.

6. Formulierung nach einem der Ansprüche 1 bis 5, **dadurch gekennzeichnet, dass** sie weiter mindestens ein pH-Wert-Einstellmittel umfasst.

7. Formulierung nach einem der Ansprüche 1 bis 6, **dadurch gekennzeichnet, dass** sie einen pH-Wert von 6 aufweist.

8. Formulierung nach einem der Ansprüche 1 bis 7, **dadurch gekennzeichnet, dass** sie eine Osmolarität zwischen 270 und 330 mOsm/kg aufweist.

9. Formulierung nach einem der Ansprüche 1 bis 8, **dadurch gekennzeichnet, dass** sie die Form einer Formulierung zur intravenösen Injektion aufweist.

10. Formulierung nach einem der Ansprüche 1 bis 9, **dadurch gekennzeichnet, dass** sie eine weitere schmerzstillende und/oder entzündungshemmende Verbindung umfasst.

11. Formulierung nach Anspruch 10, **dadurch gekennzeichnet, dass** es sich bei der schmerzstillenden Verbindung um Paracetamol handelt.

12. Sterilbeutel, der eine Formulierung nach einem der Ansprüche 1 bis 11 umfasst.

13. Verfahren zur Herstellung einer Formulierung nach einem der Ansprüche 1 bis 11, **dadurch gekennzeichnet, dass** es umfasst:
- einen Schritt des Auflösens des Ketoprofens in Wasser für Injektionszwecke, dem ein Puffermittel zugegeben wurde, und
- einen Schritt des Einstellens des pH-Wert auf einen Wert zwischen 5,5 und 6,5.

14. Herstellungsverfahren nach Anspruch 13, **dadurch gekennzeichnet, dass** es weiter einen Schritt des Sterilisierens der nach dem Schritt des Einstellens des pH-Werts erhaltenen Formulierung umfasst.

## Claims

1. Stable liquid ready-to-use ketoprofen formulation for administration by parenteral route, comprising ketoprofen solubilised in a solely aqueous solvent with a buffer agent added composed of acetic acid and sodium acetate, said formulation being adjusted to a pH above 5.5 and below 6.5, the quantity of ketoprofen being between 0.01% and 1% by weight.

2. Formulation according to claim 1, **characterised in that** the quantity of ketoprofen is 0.1% by weight.

3. Formulation according to claim 1 or 2, **characterised in that** the quantity of buffer agent is between 0.01% and 2% by weight, in particular 1.2% by weight.

4. Formulation according to any one of claims 1 to 3, **characterised in that** the ratio by weight between the acetic acid and the sodium acetate is in the range 1:20 to 1:500.

5. Formulation according to any one of claims 1 to 4, **characterised in that** the solely aqueous solvent is water for injectable preparations.

6. Formulation according to any one of claims 1 to 5, **characterised in that** it further comprises at least 1 pH-adjusting agent.

7. Formulation according to any one of claims 1 to 6, **characterised in that** it has a pH of 6.

8. Formulation according to any one of claims 1 to 7, **characterised in that** it has an osmolarity of between 270 and 330 mOsm/kg.

9. Formulation according to any one of claims 1 to 8, **characterised in that** it is in the form of a formulation for intravenous injection.

10. Formulation according to any one of claims 1 to 9, **characterised in that** it comprises another analgesic and/or anti-inflammatory compound.

11. Formulation according to claim 10, **characterised in that** the analgesic compound is paracetamol.

12. Sterile pouch comprising a formulation according to any one of claims 1 to 11.

13. Method for preparing a formulation according to any one of claims 1 to 11, **characterised in that** it comprises:
- a step of dissolving ketoprofen in water for injectable preparations with a buffer agent, and
- a step of adjusting the pH to a value of between 5.5 and 6.5.

14. Preparation method according to claim 13, **characterised in that** it further comprises a step of sterilisation of the formulation obtained after the step of adjusting the pH.
